# EUROPEAN PATENT SPECIFICATION

(11) **EP 4 110 415 B1**
(45) Date of publication and mention of the grant of the patent: **29.11.2023**
(21) Application number: 20714392.6
(22) Date of filing: 26.02.2020
(51) Int. Cl.: A61L 31/04, A61L 31/10, A61L 31/14, A61L 31/18

(54) **STENT GRAFTS HAVING A RADIOPAQUE MARKER AND METHODS OF PRODUCING**
STENTTRANSPLANTATE MIT RÖNTGENDICHTEM MARKER UND HERSTELLUNGSVERFAHREN
ENDOPROTHÈSE COUVERTE AYANT UN MARQUEUR RADIO-OPAQUE ET PROCÉDÉS DE PRODUCTION

(43) Date of publication of application: 04.01.2023
(73) Proprietor: C. R. Bard, Inc., Franklin Lakes, NJ 07417 (US)
(72) Inventor: BURGMEIER, Robert, Franklin Lakes, New Jersey 07417 (US); LECY, Cyal, Franklin Lakes, New Jersey 07417 (US)
(74) Representative: Hoffmann Eitle
(86) International application number: PCT/US2020/019827
(87) International publication number: WO 2021/173126

(56) References cited:
- WO-A1-01/56503
- WO-A2-97/37616
- DE-A1-102005 039 126
- US-A1- 2002 103 528
- US-A1- 2003 114 918

## Description

### TECHNICAL FIELD

The present specification generally relates to medical devices and, more specifically, stent grafts that include radiopaque markers.

### BACKGROUND

In the medical field, implantable prostheses are used to support vessels in a mammalian body, such as vessels in the urethra, esophagus, biliary tract, intestines, arteries, veins, and peripheral vessels. For example, implantable prostheses may be utilized to support a vessel in the mammalian body that suffers from an abnormal widening (e.g., an aneurysm, vessel contraction or lesion such as a stenosis or occlusion) or an abnormal narrowing (e.g., a stricture). In some instances, stents may be utilized. Such prostheses may include frame-like structures, such as stents, stent grafts, and covered stents.

However, conventional stents, stent grafts, and covered stents may lack the ability to be seen clearly under fluoroscopy, which can hamper the placement of the implantable prostheses. Accordingly, there are needs for radiopaque stents, stent grafts, and covered stents and methods of making radiopaque stents, stent grafts, and covered stents.

WO 01/56503 A1 discloses, according to its abstract, endoluminal prostheses and methods for their use, having discrete position indicating elements which are reported to facilitate the orienting and deploying of the prostheses within body lumens. The endoluminal prostheses may include endovascular prostheses, often formed as stent-grafts having a flexible tubular liner or "graft". The position indicating elements may include an improved radiopaque image marker to be applied to the graft, before the graft is deployed particularly within branching blood vessels for the treatment of abdominal and other aneurysms. The marker is in the form of a flat metal blank resembling a circular plate or "button". The disk may be fastened or secured onto the graft using a pair of fastening shanks or tangs which extend parallel to each other outward from a surface of the plate. WO 97/37616 A2 discloses a method for fabricating a position indicating endoluminal prosthesis e.g. a stent graft comprising providing a tubular graft comprising a polyester fabric; applying a compound to the graft, the compound comprising a polyester matrix, radiopaque particles, and a polyester solvent so that the polyester matrix adheres to the polyester fabric and permanently affixes the radiopaque particles to the graft. Suitable expansible liner materials include partially oriented polyester fibers, PTFE. To avoid marker elements which displace or otherwise interfere with the expandable frame structure, the markers will generally be applied to the polyester fabric liner as a radiopaque compound comprising radiopaque particles in a polyester matrix which is dissolved in a solvent. The radiopaque compound will wick through and bond permanently to the liner, while an overcoat will help avoiding flaking of the dried compound.

### SUMMARY

Embodiments of the present disclosure meet these needs by providing radiopaque stents, stent grafts, and covered stents and methods of making radiopaque stents, stent grafts, and covered stents.

The invention is defined by the independent claims.

According to one aspect of the present disclosure, a method of producing a radiopaque stent graft is provided. The method may include treating an exterior surface of a base stent graft to produce a functionalized surface; disposing a tie layer over the functionalized exterior surface; disposing an adherent layer over the tie layer; etching a slot into the adherent layer to produce an etched layer; treating the etched layer; and positioning a radiopaque marker in the slot to produce a filled slot and thereby produce the radiopaque stent graft.

A second aspect may include the first aspect, wherein the base stent graft comprises polytetrafluoroethylene.

A third aspect may include any of the preceding aspects, wherein the adherent layer comprises a poly(p-xylylene).

A fourth aspect may include the third aspect, wherein the poly(p-xylylene) comprises one or more of parylene C, parylene N, parylene D, parylene X, parylene AF-4, parylene SF, parylene HT, parylene VT-4 (parylene F), parylene CF, parylene A, or parylene AM.

A fifth aspect may include any of the preceding aspects, further comprising coating the filled slot with an adhesive to produce a sealant layer.

A sixth aspect may include any of the preceding aspects, further comprising etching one or more additional slots in to the adherent layer; treating the one or more additional slots; positioning a radiopaque marker into each of the one or more additional slots to produce one or more additional filled slots and thereby produce the radiopaque stent graft.

A seventh aspect may include the sixth aspect, further comprising coating the filled slots with an adhesive to produce a sealant layer.

An eighth aspect may include any of the first or fifth aspects, wherein coating the one or more additional filled slots comprises utilizing a flexible acrylate adhesive.

A ninth aspect may include the eighth aspect, further comprising curing the adhesive to produce the sealant layer.

A tenth aspect may include any of the preceding aspects, wherein etching comprises laser-etching.

An eleventh aspect may include any of the preceding aspects, wherein treating the exterior surface comprises plasma-treating the exterior surface.

An twelfth aspect may include any of the preceding aspects, wherein treating the etched layer comprises plasma-treating the etched layer.

According to a thirteenth aspect of the present disclosure, a radiopaque stent graft is provided. Embodiments of the radiopaque stent graft may include a stent graft having a functionalized surface; a tie layer surrounding a functionalized surface; and an etched layer covering at least a portion of the tie layer, wherein the etched layer comprises a filled slot, and wherein the filled slot comprises a radiopaque marker.

A fourteenth aspect may include the thirteenth aspect, further comprising a sealant layer encapsulating at least a portion of the filled slot.

A fifteenth aspect may include any of the thirteenth through fourteenth aspects, wherein the sealant layer comprises a flexible acrylate adhesive.

A sixteenth aspect may include any of the thirteenth through fifteenth aspects, wherein the base stent graft comprises polytetrafluoroethylene.

A seventeenth aspect may include any of the thirteenth through sixteenth aspects, wherein the etched layer further comprises a poly(p-xylylene).

An eighteenth aspect may include the seventeenth aspect, wherein the poly(p-xylylene) comprises one or more of parylene C, parylene N, parylene D, parylene X, parylene AF-4, parylene SF, parylene HT, parylene VT-4 (parylene F), parylene CF, parylene A, or parylene AM.

A nineteenth aspect may include any of the thirteenth through eighteenth aspects, further comprising one or more additional filled slots, wherein the one or more additional filled slots each comprise a radiopaque marker.

A twentieth aspect may include any of the thirteenth through nineteenth aspects, wherein the radiopaque marker comprises a radiopaque material.

A twenty-first aspect may include any of the thirteenth through twentieth aspects, wherein the etched adherent layer is functionalized to securely affix the radiopaque marker to the slot.

A twenty-second aspect may include any of the thirteenth through twenty-first aspects, wherein the tie layer has a thickness of from 10 angstroms to 1,000 angstroms.

These and additional features provided by the embodiments described herein will be more fully understood in view of the following detailed description, in conjunction with the drawings.

### BRIEF DESCRIPTION OF THE DRAWINGS

The embodiments set forth in the drawings are illustrative and exemplary in nature and not intended to limit the subject matter defined by the claims. The following detailed description of the illustrative embodiments can be understood when read in conjunction with the following drawings, where like structure is indicated with like reference numerals and in which:
FIG. **1A** is an illustrative depiction of a side view of a radiopaque stent graft, according to one or more embodiments shown and described herein;
FIG. **1B** is an illustrative depiction of a front view of a radiopaque stent graft, according to one or more embodiments shown and described herein;
FIG. **1C** is an illustrative depiction of a side view of a radiopaque stent graft including an optional sealant layer, according to one or more embodiments shown and described herein;
FIG. **1D** is an illustrative depiction of a front view of a radiopaque stent graft including an optional sealant layer, according to one or more embodiments shown and described herein;
FIG. **2** is an illustrative depiction of a side view of a base stent graft;
FIG. **3** is an illustrative depiction of a side view of a stent graft with a modified exterior surface;
FIG. **4** is an illustrative depiction of a side view of a stent graft with an adherent layer;
FIG. **5** is an illustrative depiction of a side view of a stent graft with an etched layer;
FIG. **6** is an illustrative depiction of a side view of a radiopaque stent graft with an etched adherent layer having filled slots, according to one or more embodiments shown and described herein;
FIG. **7** is an illustrative depiction of a side view of a radiopaque stent graft with an optional sealant layer, according to one or more embodiments shown and described herein.

### DETAILED DESCRIPTION

Embodiments as described herein are directed to radiopaque stent grafts and methods of producing a radiopaque stent grafts.

Implantable prostheses, such as stents, stent grafts, and covered stents, are currently used to treat narrowed (stenosed) tubular body vessels where the flow of blood or other biological fluids is restricted. For example, such vessels may be in the urethra, esophagus, biliary tract, intestines, arteries, veins, and peripheral vessels. Among other treatments, the implantable prostheses can be used to prop open blood vessels and reinforce collapsed or stenosed vessels in the respiratory system, reproductive system, bile or liver ducts or any other vessel.

In conventional treatment procedure methods, implantable prostheses may be implanted into the body by guiding a constraining member (i.e. a catheter) containing the implantable prosthesis to a treatment site within a body vessel. To locate the implantable prosthesis within the body vessel during deployment, the physician will generally utilize a fluoroscope, which uses X-rays, to visualize radiopaque markers on the implantable prosthesis or constraining member. However, if the radiopaque marker is located on the constraining member, and the constraining member is removed, correct placement of the implantable prosthesis cannot be confirmed. Accordingly, in order to implant the prosthesis by fluoroscopy, some portion of the implantable prosthesis should preferably be radiopaque.

However, incorporating a radiopaque marker directly into an implantable prosthesis may cause damage to the device. Under conventional methods, in order to incorporating a radiopaque marker into the device (i.e. by etching a slot in the surface of the device to receive the radiopaque marker), the modifications to the surface of the device may damage the properties of the device. For example, such conventional methods could cause the device to leak. One example includes stent grafts.

Stent grafts refer to stents used in combination with vascular grafts, which provide prostheses capable of maintaining their fit against blood vessel walls. Stent grafts may also be used in place of stents to prevent restenosis, which may occur where smooth muscle cells and other tissues can grow through a stent's mesh-like openings. Stent grafts may typically include expandable stents encapsulated within polytetrafluoroethylene (PTFE). PTFE is often used because it is biocompatible, light, porous and readily colonized by living cells. As used herein, "biocompatible" means causing little or no immunogenic reaction when placed within a mammalian body. Although there are advantages to using stent grafts comprising PTFE, the PTFE surface itself is not reactive. Accordingly, it may be nearly impossible to attach radiopaque markers to the PTFE surface of conventional stent grafts without damaging the stent graft.

Accordingly there are needs for radiopaque stent grafts and improved methods of making radiopaque stent grafts. Moreover there are needs for methods of making radiopaque stent grafts that may cause little or no damage to the stent graft. Embodiments of the radiopaque stent grafts described herein may be useful to confirm the correct placement of the stent within the vessel during a treatment procedure. Accurate visualization of the radiopaque stent grafts described herein may minimize the invasiveness of the procedure and allow for further visualization during follow-up inspections.

### Radiopaque Stent Grafts

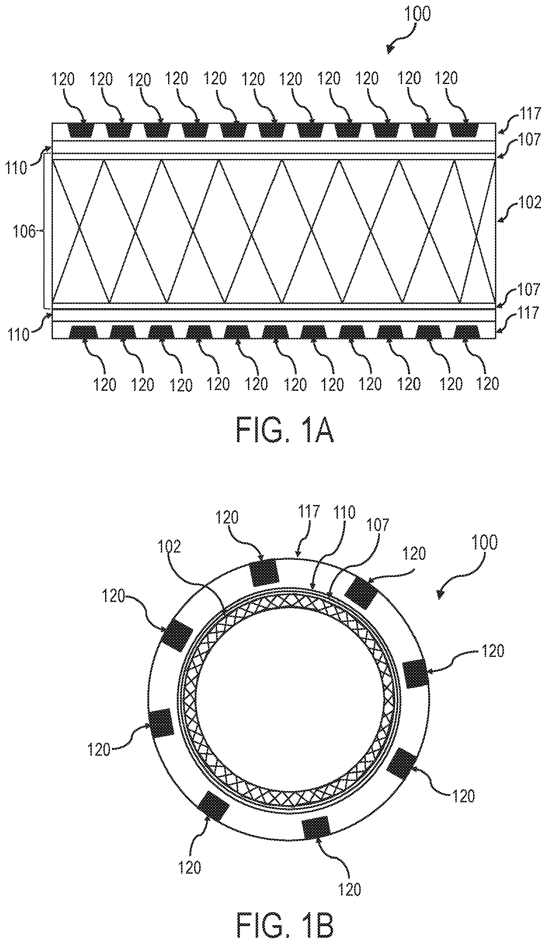

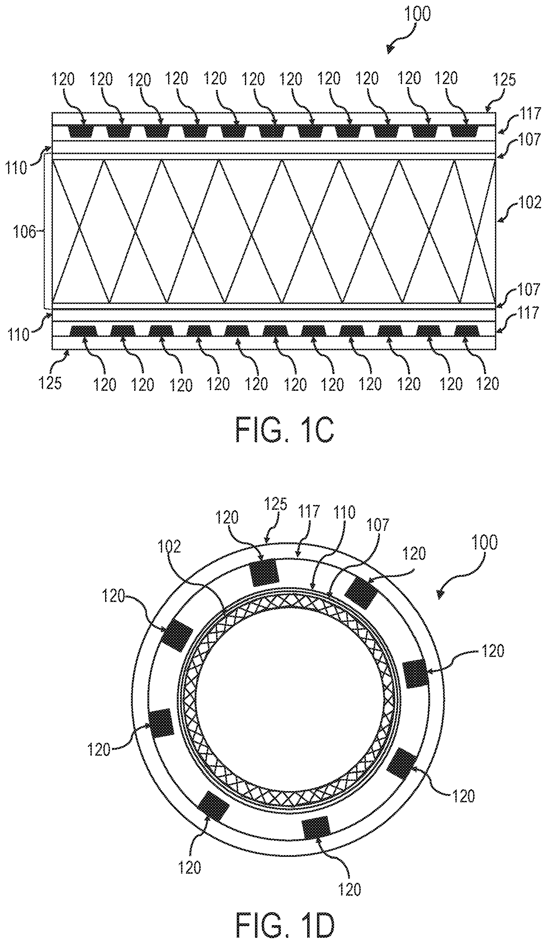

Embodiments of radiopaque stents grafts will now be described. FIGS. **1A** and FIG. **1B** are illustrative depictions of a side view and front view, respectively, of a radiopaque stent graft **100,** according to one or more embodiments shown and described herein. FIGS. **1C** and FIG. **1D** are illustrative depictions of a side view and front view, respectively, of a radiopaque stent graft **100** with an optional sealant layer, according to one or more embodiments shown and described herein.

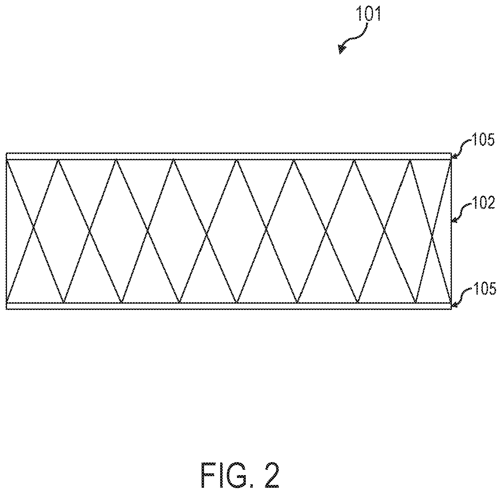

Referring to FIGS. **1A-1D** and FIG. **2****,** in embodiments, the radiopaque stent graft **100** may include a stent graft **106** with a functionalized surface **107.** The stent graft **106** may be produced from a base stent graft **101,** such as the one shown in FIG. **2****,** having an expandable stent **102** encapsulated within a vascular graft **105.**

Referring still to FIG. **2****,** the expandable stent **102** of the base stent graft **101** may be a balloon expanded or self-expanding stent. The expandable stent **102** may be made by various manufacturing methods, including forming wire and machining a hollow tube. Machining methods may include one or more of photo-chemical etching, laser-cutting, stamping, piercing, or other material-removal processes. Other manufacturing methods may include vacuum or chemical deposition of material or forming a tube of machined flat material.

In embodiments, the expandable stent **102** of the base stent graft **101** may be configured as a series of rings connected together to form a lattice-like framework thereby defining a tubular framework. In embodiments, the series of rings may or may not be essentially identical. In embodiments, the rings may be arranged in a circular configuration, a helical configuration, or a combination of helical and circular frameworks. In embodiments, the series of rings may or may not have connecting linkages between the adjacent rings. In embodiments that do not utilize connecting linkages between adjacent rings, the series of rings may be connected by a direct connection between one ring and the next ring. The series of rings may be placed adjacent to each other in order to maintain an appropriate longitudinal spacing between each rings.

The expandable stent **102** of the base stent graft **101** may comprise a biocompatible metal alloy. Examples of biocompatible metal alloys may include stainless steel, Nitinol or Elgiloy. In embodiments, the shape memory characteristics of Nitinol may allow the expandable stent **102** of the base stent graft **101** to self-expand when placed in a tubular body vessel at normal body temperature.

In embodiments, the vascular graft **105** of the base stent graft **101** may comprise PTFE, polyvinyl chloride (PVC), polyethylene terephthalate (PET), polyethylene, Nylon, PEBAX (i.e. a copolymer of polyether and polyamide), polystyrene (PS), polyethleneterephthalate (PETP), Dacron mesh reinforced umbilical tissues, bovine collagen, polyester knitted collagen, tricot knitted polyester collagen impregnated, polyurethane (available under the trade mark "Vectra R") or various other suitable materials as will be apparent to those of ordinary skill in the art. In embodiments, the base stent graft **101** may be a self-expanding stent encapsulated within PTFE. In embodiments, the base stent graft **101** may be a self-expanding Nitinol stent encapsulated within PTFE. One example is known commercially as "Fluency^{®}," which is marketed by C.R. Bard Peripheral Vascular Inc.

In embodiments, the thickness of the vascular graft **105,** the "wall thickness," may provide flexibility to the base stent graft **101** to help assist manipulation of the radiopaque stent graft **100** during implantation. In embodiments, wall thickness of the vascular graft **105** may affect the overall size of the radiopaque stent graft **100** and the associated delivery system. For example, in embodiments, a relatively thinner wall thickness may allow for a relatively smaller radiopaque stent graft **100** to be manufactured, which may be delivered using a relatively smaller size catheter based delivery system. Additionally, wall thickness may affect the structural integrity of the radiopaque stent graft **100.** For example, a thin wall thickness may be prone to structural degradation or kinking during implantation.

In embodiments, the wall thickness of the vascular graft **105** may range from about 50 micrometers (microns) to about 1000 microns. In embodiments, the wall thickness of the vascular graft **105** may range from about 50 microns to about 800 microns, from about 50 microns to about 600 microns, from about 50 microns to about 400 microns, from about 50 microns to about to about 200 microns, from about 50 microns to about 100 microns, from about 100 microns to about 1000 microns, from about 100 microns to about 800 microns, from about 100 microns to about 600 microns, from about 100 microns to about 400 microns, from about 100 microns to about to about 200 microns, from about 200 microns to about 1000 microns, from about 200 microns to about 800 microns, from about 200 microns to about 600 microns, from about 200 microns to about 400 microns, from about 400 microns to about 1000 microns, from about 400 microns to about 800 microns, from about 400 microns to about 600 microns, from about 600 microns to about 1000 microns, from about 600 microns to about 800 microns, or from about 800 microns to about 1000 microns.

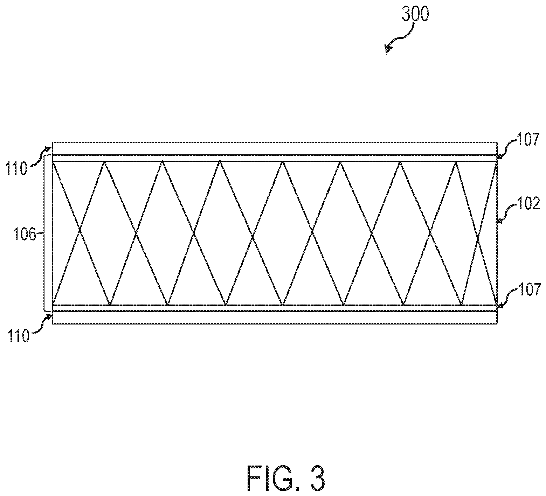

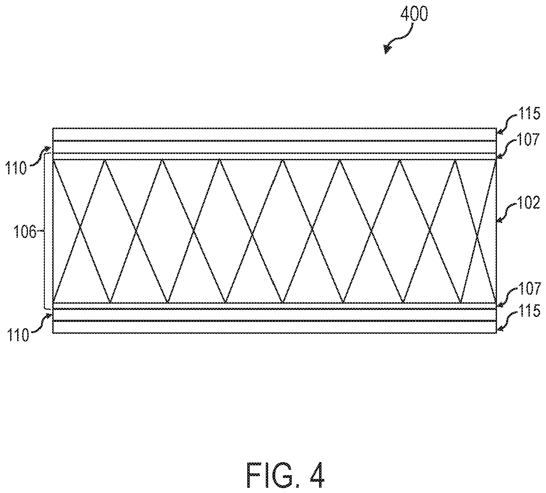

Referring now to FIGS. **3-4****,** the radiopaque stent graft **100** may include a functionalized surface **107,** namely, a surface that has been subjected to a surface modification that decreases a surface free energy of the exterior surface of the vascular graft **105** before application of an adherent layer **115** (as shown in FIG. **4** and discussed in more detail below). As will be described below in more detail, the surface modification may include plasma treatment of the exterior surface of the vascular graft **105.**

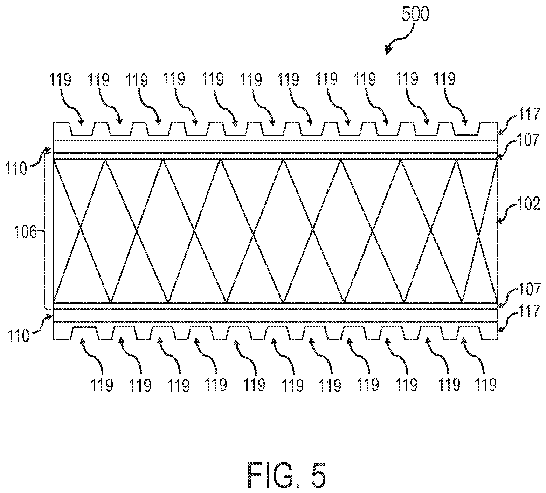

Referring now to FIGS. **5-6****,** in embodiments, the etched layer **117** may further include one or more slots **119,** where "slots" may refer to depots or surface features formed by etching the adherent layer **115.** Subsequently one or more radiopaque markers are applied to the one or more slots **119** thereby producing one or more filled slots **120.** The radiopaque markers may fill at least a portion of the depots or surface features in the etched layer **117.** Accordingly, the one or more filled slots **120** comprising one or more radiopaque markers allow for fluoroscopic visualization of the radiopaque stent graft **100** during implantation. In embodiments, the one or more radiopaque markers have radiopaque features incorporated therein, which can be used as visual markers located by fluoroscopy.

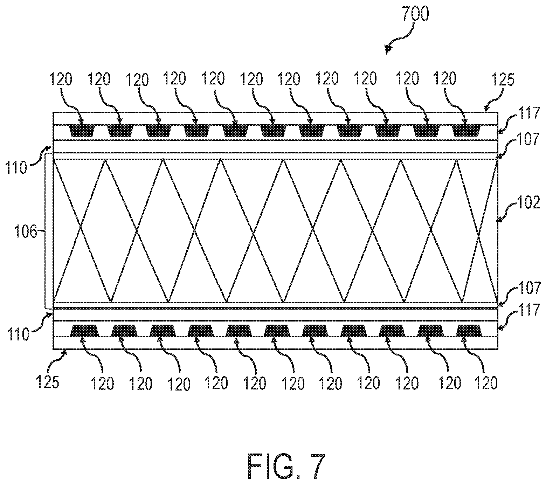

Referring now to FIG. **7****,** because the radiopaque stent graft **100** may be visualized by fluoroscopy during implementation, deployment of the presently-disclosed radiopaque stent grafts at unintended locations may be prevented. Additionally, the invasiveness associated with multiple deployment attempts or relocation may be reduced or eliminated. In embodiments, visualization of the radiopaque stent graft **100** may be utilized for both implantation and follow-up inspection during treatment.

In some embodiments, the radiopaque stent graft **100** may include an sealant layer **125,** which includes an adhesive coated over the one or more filled slots **120.**

### Methods of Producing Radiopaque Stent Grafts

Embodiments of methods of producing radiopaque implantable prostheses will now be described. In embodiments, the disclosed methods may produce radiopaque stent grafts, as previously described in this disclosure. Although, the following method will be described as a method of producing a radiopaque stent graft, it should be understood that other implantable prostheses are also contemplated.

Referring now generally to FIGS. **2-7****,** embodiments of the method of producing a radiopaque stent graft **100** may include functionalizing the exterior surface of the vascular graft **105** of a base stent graft **101** to produce a stent graft **106** with a functionalized surface **107;** disposing a tie layer **110** over the functionalized surface **107;** disposing an adherent layer **115** over the tie layer **110;** etching one or more slots **119** into the adherent layer **115;** functionalizing the one or more slots **119;** and positioning a radiopaque marker in the one or more slots **119** to produce one or more filled slots **120** and thereby produce the radiopaque stent graft **100.**

As stated previously, FIG. **2** is an illustrative depiction of a side view of a base stent graft **101.** In embodiments, the base stent graft **101** includes a vascular graft **105,** which has an exterior surface. The exterior surface of the vascular graft **105** may be inert. In embodiments, the exterior surface of the vascular graft **105** of the base stent graft **101** may be subjected to an exemplary surface modification process to functionalize the exterior surface of the vascular graft **105** and thereby produce a stent graft **106** with a functionalized surface **107,** as shown in FIG. **3****.** Referring to FIGS. **2-3****,** the surface modification may increase the functionality of the exterior surface of the vascular graft **105** producing a functionalized surface **107** that one or more layers may be applied to the stent graft **106,** as described below.

Referring back to FIGS. **1A-1D****,** and as previously described, the radiopaque stent graft **100** may include a functionalized surface **107,** namely, a surface that has been subjected to a surface modification. Referring again to FIGS. **2-3****,** In embodiments, the surface modification may include plasma treatment of the exterior surface of the vascular graft **105** of the base stent graft **101.** In embodiments, an exterior surface of the vascular graft **105** is subjected to an exemplary plasma treatment process to functionalize the exterior surface of the vascular graft **105** and thereby produce a functionalized surface **107.** As stated previously, in embodiments, the vascular graft **105** may be formed of a polymer material. In exemplary embodiments, the vascular graft **105** may be formed of a polymer material that comprises polytetrafluoroethylene (PTFE), polyvinyl chloride (PVC), polyethylene terephthalate (PET), polyethylene, Nylon, PEBAX (i.e. a copolymer of polyether and polyamide), polystyrene (PS), polyethleneterephthalate (PETP), or various other suitable materials as will be apparent to those of ordinary skill in the art. The plasma treatment process may increase the functionality of the exterior surface of the vascular graft **105,** thereby producing functionalized surface **107** so that one or more layers may be applied to the stent graft **106.**

Referring still to FIGS. **2-3****,** In embodiments, the plasma treatment process is configured to functionalize the base stent graft **101** along the exterior surface of the vascular graft **105** and/or other portions of the of the vascular graft **105.** By functionalizing the exterior surface of the vascular graft **105** prior to the application of the adherent layer **115** (as shown in FIG. 4 described subsequently) onto the base stent graft **101,** the plasma treatment process is operable to modify the material properties of the exterior surface of the vascular graft **105** to thereby provide improved interactions between the adherent layer **115** and the functionalized surface **107.** For example, treating the exterior surface of the vascular graft **105** with the plasma treatment process allows for the positioning of one or more radiopaque markers onto the stent graft **106** (via the adherent layer) without damaging the stent graft **106.**

Referring still to FIGS. **2-3****,** In exemplary use, at least a portion of the base stent graft **101** is positioned within a plasma chamber (not shown) for treating pursuant to the plasma treatment process. In the present example, the base stent graft **101** is positioned within the plasma chamber such that exterior surface of the vascular graft **105** is treated by the plasma treatment process. Although the treatment of the exterior surface of the vascular graft **105** is described in the present embodiments, it should be understood that other various portions of the base stent graft **101** and/or other suitable implantable prostheses may be treated with plasma treatment process of the present disclosure as will be apparent to those of ordinary skill in the art. With the base stent graft **101** positioned therein, the plasma treatment process commences by generating a desired vacuum within the chamber. In this instance, the exterior surface of the vascular graft **105** is subjected to the vacuum atmosphere generated within the chamber. During the plasma treatment, the temperatures and pressures used within the chamber may depend on the materials of the base stent graft **101.** For example, too high of a temperature may destroy materials of the base stent graft **101.** In embodiments, the temperature within the chamber may be from 25°C to 50°C, from 20°C to 40°C, from 20°C to 30°C, from 30°C to 50°C, from 30°C to 40°C, or from 40°C to 50°C.

With the vacuum pressure maintained within the chamber, the polymer material of the exterior surface of the vascular graft **105** is exposed to a gas supplied by a gas source (not shown) coupled to the plasma chamber. In embodiments, the exterior surface of the vascular graft **105** may be exposed to xenon, argon, fluorine, a fluorocarbon gas mixture, a xenon/oxygen gas mixture, or other suitable gas mixture supplied by the gas source. In specific embodiments, the exterior surface of the vascular graft **105** may be exposed to xenon, argon, or a xenon/oxygen gas mixture supplied by the gas source. Depending on the type of gas supplied, the gas source is operable to generate ions (i.e. fluorinated ions) and radicals from the gas transmitted into the chamber. In some embodiments, the gas source is operable to generate fluorine-containing ions (i.e. fluorinated ions) and radicals from the fluorine or fluorocarbon gas transmitted into the chamber. By way of example only, the gases that may be transmitted by the gas source may comprise tetrafluoromethane (CF₄), hexafluoroethane (C₂F₆), xenon difluoride (XeF₂), fluorine (F₂), chloropentafluoroethane (CF₃CCIF₂), sulfur hexafluoride (SF₆), and other suitable gases as will be apparent to those of ordinary skill in the art. The discharge of the mixture into the chamber subjects the exterior surface of the vascular graft **105** to the gas at a predetermined flow and for a predetermined duration.

In embodiments, then, a plasma generator (not shown) that is in fluidic communication with the chamber is activated to thereby discharge plasma into the container and thereby expose the exterior surface of the vascular graft **105** to the plasma. As merely an illustrative example, the plasma generator may comprise an arc discharger, a dielectric barrier discharger, a spark discharger, resistive barrier dischargers, radio-frequency excitation, microwave frequency excitation, and other suitable generators as will be apparent to those of ordinary skill in the art.

The exterior surface of the vascular graft **105** may be treated with the plasma sourced from the plasma generator such that the plasma interacts with the polymer material of the exterior surface of the vascular graft **105** at a predetermined flow. In embodiments, the generated plasma comprises ions and radicals. The plasma ion-implantation step may comprise initially extracting ions from the plasma generator and subsequently selecting desired ions for transmission through an accelerating column via a magnetic field (not shown) of the plasma generator. In this instance, the ions selected by the magnetic field are forced into the polymer material of the exterior surface of the vascular graft **105** such that the ions are effectively implanted into the of the vascular graft **105,** thereby producing the functionalized surface **107.** Hydrogen atoms of the polymer material of the exterior surface of the vascular graft **105** are simultaneously replaced by the ion species implanted thereon.

In an exemplary embodiment, the exterior surface of the vascular graft **105** may be fluorinated with the plasma sourced from the plasma generator such that the plasma interacts with the polymer material of the exterior surface of the vascular graft **105** at a predetermined flow. In the present example, the generated plasma comprises fluorine-containing ions and radicals. The plasma ion-implantation step may comprise initially extracting fluorine-containing ions from the plasma generator and subsequently selecting desired ions for transmission through an accelerating column via a magnetic field (not shown) of the plasma generator. In this instance, the fluorine-containing ions selected by the magnetic field are forced into the polymer material of the exterior surface of the vascular graft **105** such that the ions are effectively implanted into the exterior surface of the vascular graft **105.** Hydrogen atoms of the polymer material of the exterior surface of the vascular graft **105** are simultaneously replaced by the fluorinated species implanted thereon.

In embodiments, then, the portion of the base stent graft **101** contained within the plasma chamber (i.e. the exterior surface of the vascular graft **105**) is continuously treated with the plasma for a predetermined duration, thereby forming a functionalized surface **107** along the stent graft **106.** The plasma treatment process may be concluded once the exterior surface of the vascular graft **105** has been exposed to the plasma for the predetermined duration, resulting in the functionalized surface **107.**

Referring to FIG. **3****,** in embodiments, after producing the functionalized surface **107** a tie layer **110** may be disposed over the functionalized surface **107.** The term "tie layer" as used herein, refers to a layer that adheres two layers together. For example a tie layer may bond polar materials to one or more layers that do not include polar materials. In embodiments, the tie layer may include any material capable of adhering the functionalized surface **107** to the subsequently applied adherent layer **115,** which is described in more detail below. In some embodiments, the tie layer **110** may be a silane layer.

In embodiments, application of the tie layer **110** may be applied by various methods known in the art. For example, the tie layer **110** may be coated onto functionalized surface **107** by various coating methods known in the art, for example, by dipping, spraying, inkjet printing, other methods, or combinations. In other embodiments, the tie layer **110** may be applied onto functionalized surface **107** by a plasma treatment process. The plasma treatment process may be selected based on the materials of the tie layer **110.** In some embodiments, the plasma treatment method applying the tie layer **110** onto functionalized surface **107** may not utilize an electric field within the plasma chamber. In embodiments, the tie layer **110** may be applied onto functionalized surface **107** by a plasma treatment process, which is performed in the same plasma chamber as previously described. Alternatively or additionally, the tie layer **110** may be applied onto functionalized surface **107** outside of the plasma chamber previously described or in a different plasma chamber. Embodiments of applying the tie layer **110** onto functionalized surface **107** may further include combinations of coating methods and plasma treatment methods.

As seen in FIG. **3****,** the tie layer **110** disposed onto the functionalized surface **107** may be relatively minimal and include a substantially uniform distribution along the surface area of the functionalized surface **107.** By way of example only, the thickness of tie layer **110** implanted onto the functionalized surface **107** is less than one micron. In embodiments, the tie layer **110** may be from 10 angstroms (Å) to 1000 Å, from 10 Å to 750 Å, from 10 Å to 500 Å, from 10 Å to 250 Å, from 10 Å to 100 Å, from 100 Å to 1000 Å, from 100 Å to 750 Å, from 100 Å to 500 Å, from 100 Å to 250 Å, from 250 Å to 1000 Å, from 250 Å to 750 Å, from 250 Å to 500 Å, from 500 Å to 1000 Å, from 500 Å to 750 Å, or from 750 Å to 1000 Å.

With the plasma treatment process producing the functionalized surface **107** being complete and the tie layer **110** disposed thereon being complete; the treatment process effectively forms a surface upon which an adherent layer may be disposed. As should be understood in view of the teachings herein, optimal results may be achieved during the plasma treatment process by applying appropriate gas types, gas flow, treatment times, and generator power.

With the minimal thickness of the tie layer **110** disposed onto the functionalized surface **107,** an adherent layer **115** is disposed onto the tie layer **110,** as seen in FIG. **4****.** In embodiments, the adherent layer **115** allows for the positioning of one or more radiopaque markers onto the stent graft **106** (via the adherent layer) without damaging the vascular graft of the stent graft **106.**

The adherent layer **115** may include a polymer. Particularly suitable polymers of the adherent layer **115** include biocompatible polymers that avoid undesirable irritation of body tissue. Example polymers include polymers formed from cycloaliphatic monomers or aromatic monomers. Examples of cycloaliphatic monomers include alkylcyclohexanes such as methylcyclohexane. Examples of aromatic monomers include alkylbenzenes such as toluene and xylenes. In some embodiments, the intermediate layer may be a poly(p-xylylene) such as parylene C, parylene N, parylene D, parylene X, parylene AF-4, parylene SF, parylene HT, parylene VT-4 (parylene F), parylene CF, parylene A, or parylene AM, for example. Structures of selected parylenes are provided below:

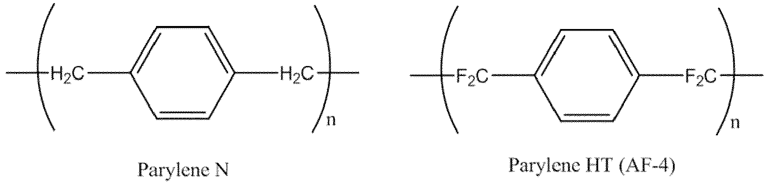

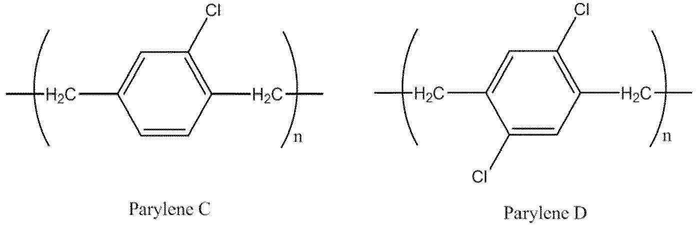

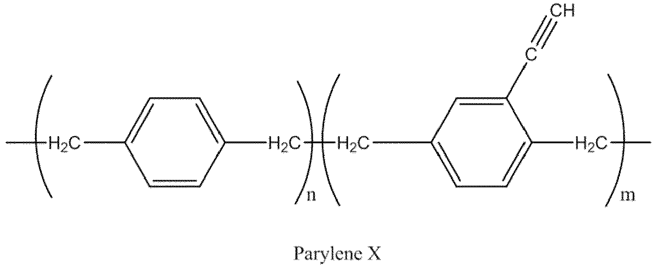

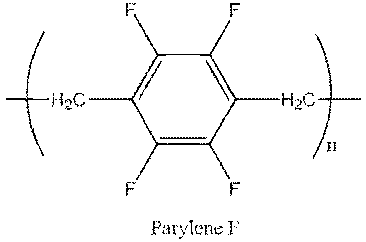

Additional polymers may be present in the adherent layer **115.** Examples of such additional polymers include, for example, polyolefins, polyisobutylene, ethylene-α-olefin copolymers, acrylic polymers and copolymers, polyvinyl chloride, polyvinyl methyl ether, polyvinylidene fluoride and polyvinylidene chloride, polyacrylonitrile, polyvinyl ketones, polystyrene, polyvinyl acetate, ethylene-methyl methacrylate copolymers, acrylonitrile-styrene copolymers, ABS resins, Nylon 12 and its block copolymers, polycaprolactone, polyoxymethylenes, polyethers, epoxy resins, polyurethanes, rayon-triacetate, cellulose, cellulose acetate, cellulose butyrate, cellophane, cellulose nitrate, cellulose propionate, cellulose ethers, carboxymethyl cellulose, chitins, polylactic acid, polyglycolic acid, polylactic acid-polyethylene oxide copolymers, polyethylene glycol, polypropylene glycol, polyvinyl alcohol, and mixtures and block copolymers thereof. In embodiments, the additional polymers may be chosen from polymers having a low surface free-energy.

Without intent to be bound by theory, it is believed that adherent layers including certain polymer materials such as parylenes, for example, may adhere to the functionalized surface **107** via the tie layer **110**) and thereby produce a layer upon which one or more radiopaque markers may be applied to the stent graft **106.**

Because the implantable prostheses according to embodiments, particularly stent grafts, for example, undergo mechanical manipulation, i.e., expansion and contraction, further examples of polymers that may be useful in the adherent layer **115** may include elastomeric polymers, such as silicones (e.g., polysiloxanes and substituted polysiloxanes), polyurethanes, thermoplastic elastomers, ethylene vinyl acetate copolymers, polyolefin elastomers, and EPDM rubbers. Because of the elastic characteristics of these polymers, when these polymers are included in the adherent layer **115,** adherence of the radiopaque markers to the surface of the adherent layer **115** and ultimately to the implantable prostheses may increase when the implantable prostheses is subjected to forces or stress.

The adherent layer **115** may comprise one or more other components in order to maintain the integrity and adherence of the one or more radiopaque markers and/or sealant layers to the implantable prostheses, to facilitate both adherence of radiopaque markers and additive components during transit and deployment at the treatment site within the body vessel, or combinations of these benefits.

In embodiments, the adherent layer **115** may have a thickness from 1 microns to 5 microns, from 1 microns to 4 microns, from 1 microns to 3 microns, from 1 microns to 2 microns, from 2 microns to 5 microns, from 2 microns to 4 microns, from 2 microns to 3 microns, from 3 microns to 5 microns, from 3 microns to 4 microns, or from 4 microns to 5 microns.

Referring now to FIG. **5****,** in embodiments, one or more slots may be etched into the adherent layer **115** thereby producing an etched layer **117.** As used herein, "etching" means creating a surface modification in the surface of the adherent layer **115.** The etching methods may be used to etch one or more slots **119** into the adherent layer **115** to produce the etched layer **117.** The one or more slots **119** may allow for improved adherence of the radiopaque markers within the adherent layer **115.**

Various etching methods are contemplated, which may include utilizing chemical methods, mechanical methods, lasers, other etching methods, and combinations thereof. In embodiments, etching may include utilizing solvents to selectively dissolve the surface of the adherent layer **115.** In embodiments, etching may include utilizing a laser. In further embodiments, the laser may be an excimer laser. In embodiments, etching may include utilizing a mechanical etching device, such as blades or shears. Selection of a particular etching method may depend on desired efficiency of the overall process and/or the desired precision of the method. In a specific embodiment, an excimer laser etching may be selected to produce one more slots with precise dimensions.

The size and number of the one or more slots **119** may depend on the desired radiopacity of the radiopaque stent graft **100.** In some embodiments, only one slot may be desired. In other embodiments, one or more additional slots may be desired. In embodiments, the one or more slots may be etched in any orientation or in any pattern along the surface of the adherent layer **115.** For example, in embodiments, the one or more slots **119** may be etched in radial directions, longitudinal directions, or both.

Referring still to FIG. **5****,** in embodiments, the etched layer **117** may be subjected to a second plasma treatment process. The second plasma treatment process may allow for improved adherence of the one or more radiopaque markers onto the etched layer **117** (via the one or more slots **119**). As previously explained, the etched layer **117** is formed of a polymer. Applying the second plasma treatment process to the polymer material of the etched layer **117** may functionalize the etched layer **117.**

In the present example, the second treatment process may be configured to improve the adherence properties of the etched layer **117.** By functionalizing the surface of the etched layer **117** prior to the application of one or more radiopaque markers into the one or more slots **119,** the second plasma treatment process may modify the material properties of the etched layer **117** to thereby provide improved interactions between the one or more radiopaque markers and the etched layer **117.** For example, treating the surface of the etched layer **117** with the second plasma treatment process may allow for the one or more radiopaque markers to adhere to the etched layer **117.** Ultimately, the second plasma treatment process may be operable to provide a radiopaque stent graft **100** by a method that prevents damage to the stent graft **106.**

In exemplary use, at least a portion of the etched layer **117** may be positioned within a second plasma chamber (not shown) for treating pursuant to the second plasma treatment process. In the present example, at least a portion of the etched layer **117** may be positioned within the plasma chamber such that the etched layer **117** is treated by the second plasma treatment process. With at least a portion of the etched layer **117** positioned therein, the second treatment process may commence by generating a desired vacuum within the second plasma chamber. In this instance, the etched layer **117** may be subjected to the vacuum atmosphere generated within the chamber. The temperature within the chamber may depend on the materials of the etched layer **117.** In embodiments, the temperature within the chamber may be from 25°C to 50°C, from 20°C to 40°C, from 20°C to 30°C, from 30°C to 50°C, from 30°C to 40°C, or from 40°C to 50°C.

With the vacuum pressure maintained within the chamber, the polymer material of the exterior surface of the vascular graft **105** is exposed a gas supplied by a gas source (not shown) coupled to the plasma chamber. In embodiments, the etched layer **117** may be exposed to xenon, argon, fluorine, a fluorocarbon gas mixture, a xenon/oxygen gas mixture, an acrylate gas or other suitable gas mixture supplied by the gas source. In specific embodiments, the etched layer **117** may be exposed to a xenon and acrylate gas mixture supplied by the gas source. Depending on the type of gas supplied, the gas source is operable to ions (i.e. fluorinated ions) and radicals from the gas transmitted into the chamber. In some embodiments, the gas source is operable to generate fluorine-containing ions (i.e. fluorinated ions) and radicals from the fluorine or fluorocarbon gas transmitted into the chamber. By way of example only, the gases that may be transmitted by the gas source may comprise tetrafluoromethane (CF₄), hexafluoroethane (C₂F₆), xenon difluoride (XeF₂), fluorine (F₂), chloropentafluoroethane (CF₃CCIF₂), sulfur hexafluoride (SF₆), and other suitable gases as will be apparent to those of ordinary skill in the art. The discharge of the mixture into the chamber subjects the etched layer **117** to the gas at a predetermined flow and for a predetermined duration.

In embodiments, then, a second plasma generator (not shown) that is in fluidic communication with the chamber may be activated to thereby discharge plasma into the container and thereby expose the etched layer **117** to the plasma. As merely an illustrative example, the second plasma generator may comprise an arc discharger, a dielectric barrier discharger, a spark discharger, resistive barrier dischargers, radio-frequency excitation, microwave frequency excitation, and other suitable generators as will be apparent to those of ordinary skill in the art.

The etched layer **117** may be fluorinated with the plasma sourced from the second plasma generator such that the plasma interacts with the polymer material of the etched layer **117** at a predetermined flow. In the present example, the generated plasma comprises ions and radicals. The plasma ion-implantation step may comprise initially extracting ions from the second plasma generator and subsequently selecting desired ions for transmission through an accelerating column via a magnetic field (not shown) of the second plasma generator. In this instance, the ions selected by the magnetic field are forced into the polymer material of the etched layer **117** such that the ions are effectively implanted into the etched layer **117.** Hydrogen atoms of the polymer material of the etched layer **117** are simultaneously replaced by the ion species implanted thereon.

In embodiments, then, the portion of the etched layer **117** contained within the second plasma chamber may be continuously treated with the plasma for a predetermined duration, thereby forming a functionalized surface along the etched layer **117.** The second plasma treatment process may be concluded once the etched layer **117** has been exposed to the plasma for a predetermined duration, resulting in the etched layer **117** comprising a plasma layer (not shown) implanted thereon, thereby functionalizing the etched layer **117.**

In embodiments, a functionalized plasma layer (not shown) may be implanted onto the etched layer **117,** which may be relatively minimal and may include a substantially uniform distribution along the surface area of the etched layer **117.** By way of example only, the thickness of plasma layer implanted onto the etched layer **117** may be less than one micron. With the reaction of the polymer surface of the etched layer **117** and plasma layer applied thereon being complete; the second plasma treatment process effectively forms a functionalized surface upon which the one or more radiopaque markers may be applied with improved adherence. As should be understood in view of the teachings herein, optimal results may be achieved during the second plasma treatment process by applying appropriate gas types, gas flow, treatment times, and generator power. With the minimal thickness of the plasma layer applied onto the etched layer **117,** the one or more radiopaque markers may be applied into one or more slots **119** of the etched layer **117.**

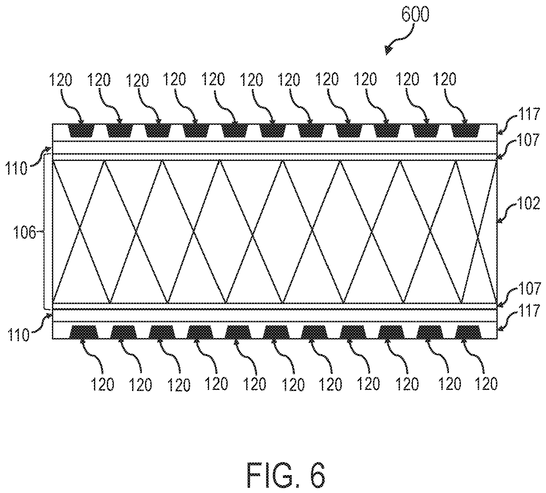

Referring now to FIG. **6****,** in embodiments, a radiopaque marker may be positioned in each of the one or more slots **119,** producing filled slots **120,** and thereby producing a radiopaque stent graft **100.** The radiopaque markers may fill at least a portion of the one or more slots **119** in the etched layer **117.** Accordingly, the one or more filled slots **120** comprising one or more radiopaque markers allow for fluoroscopic visualization of the radiopaque stent graft **100** during implantation. In embodiments, the one or more radiopaque markers have radiopaque features incorporated therein, which can be used as visual markers located by fluoroscopy.

The radiopaque markers may comprise a radiopaque material. For example, radiopaque materials may include polymers, gold, platinum-iridium alloy, bismuth subcarbonate, barium sulfate, bismuth oxychloride, bismuth trioxide, tungsten, combinations, and various other radiopaque materials know to those skilled in the art.

Referring now to FIG. **7****,** in embodiments of the radiopaque stent graft **100,** the etched layer **117** having filled slots **120** may be optionally coated with an adhesive, producing a sealant layer **125.** The sealant layer **125** may encapsulate the one or more radiopaque markers within the one or more filled slots **120** to seal the one or more radiopaque markers within the one or more filled slots **120** of the etched layer **117.** In embodiments, the sealant layer **125** may fully encapsulate the one or more radiopaque markers within the one or more filled slots **120** to prevent the one or more radiopaque markers from being exposed to the surrounding environment for safety.

The sealant layer **125** may comprise an adhesive that has been coated onto the etched layer **117** having filled slots **120.** The adhesive utilized may be selected based on the radiopaque material selected above and other properties, including biocompatibility. In embodiments, adhesives used to produce the sealant layer **125** may include flexible acrylate adhesives.

The adhesive may be coated onto the etched layer **117** having filled slots **120** by various coating methods known in the art. In embodiments, the adhesive may be coated onto the etched layer **117** having filled slots **120** by dipping, spraying, or other methods. In embodiments, the method may further comprise curing the adhesive to form the sealant layer **125.** In embodiments, curing the adhesive may including exposing the adhesive to a ultraviolet light emitted by an ultraviolet light source (not shown). In embodiments, the adhesive may be exposed to the ultraviolet light at an intensity and for a duration sufficient to completely cure the adhesive and thereby produce the sealant layer **125.** In further embodiments, one or more additional layers may be applied, for example, to improve the biocompatibility of the radiopaque stent graft **100.**

### Treatment Methods Using the Radiopaque Stent Grafts

Embodiments will now be described for treatment methods that utilizing the radiopaque stent grafts described herein.

In embodiments, the radiopaque stent graft **100** may be delivered via a small incision on a mammalian body. In embodiments, the radiopaque stent graft **100** may be delivered to a damaged or diseased site (the treatment site) via a constraining member. Constraining members may include a catheter or sheath. In embodiments, the constraining member and the radiopaque stent graft **100** may together be inserted into the small incision, guided through the body vessel to the treatment site, and deployed by removing the constraining member. In embodiments, the radiopaque stent graft **100** may then self-expand at normal body temperature once placed at the treatment site and after the constraining member has been removed.

To direct the radiopaque stent graft **100** to the precise location of the treatment site, the radiopaque markers of the radiopaque stent graft **100** may be visualized using a fluoroscope by means of X-ray. In embodiments, the radiopaque stent graft **100** may be used to confirm the correct placement within the body vessel. The radiopaque stent graft **100** may be used to visually verify the orientation of the radiopaque stent graft **100** to determine whether the radiopaque stent graft **100** has been mislocated, twisted, or kinked. Utilizing the radiopaque stent graft **100** described herein may reduce the risk of deployment of the radiopaque stent graft **100** at an unintended location, which could result in immediate trauma. Utilizing the radiopaque stent graft **100** described herein may reduce the risk of increased invasiveness associated with multiple deployment attempts and relocation of the constraining member.

It should now be understood that embodiments as described herein are directed the systems, methods, and catheters for treatment of a body vessel. In particular, embodiments as described herein include radiopaque stent grafts that may allow for visualization and therefore provide treatment methods that are minimally invasive.

It is noted that the terms "substantially" and "about" may be utilized herein to represent the inherent degree of uncertainty that may be attributed to any quantitative comparison, value, measurement, or other representation. These terms are also utilized herein to represent the degree by which a quantitative representation may vary from a stated reference without resulting in a change in the basic function of the subject matter at issue.

## Claims

1. A method of producing a radiopaque stent graft (100) comprising:
treating an exterior surface of a base stent graft (101) to produce a functionalized surface (107);
disposing a tie layer (110) over the functionalized surface (107);
disposing an adherent layer (115) over the tie layer (110);
etching a slot (119) into the adherent layer (115) to produce an etched layer;
treating the etched layer; and
positioning a radiopaque marker in the slot (119) of the etched layer to produce a filled slot and thereby produce the radiopaque stent graft (100).

2. The method of claim 1, wherein the base stent graft (101) comprises polytetrafluoroethylene.

3. The method of any preceding claim, wherein the adherent layer (115) comprises a poly(p-xylylene), wherein the poly(p-xylylene) preferably comprises one or more of parylene C, parylene N, parylene D, parylene X, parylene AF-4, parylene SF, parylene HT, parylene VT-4 (parylene F), parylene CF, parylene A, or parylene AM.

4. The method of any preceding claim, further comprising coating the filled slot with an adhesive to produce a sealant layer (125).

5. The method of any preceding claim, further comprising:
etching one or more additional slots (119) in to the adherent layer;
plasma-treating the one or more additional slots (119);
positioning a radiopaque marker into each of the one or more additional slots (119) to produce one or more additional filled slots (119) and thereby produce the radiopaque stent graft, preferably further comprising coating the filled slots with an adhesive to produce a sealant layer (125), wherein coating the one or more additional filled slots (119) more preferably comprises utilizing a flexible acrylate adhesive, further even more preferably comprising curing the adhesive to produce the sealant layer.

6. The method of any preceding claim, wherein etching comprises laser-etching and/or wherein treating the exterior surface of a base stent graft (101) comprises plasma-treating the exterior surface.

7. The method of any preceding claim, wherein treating the etched layer comprises plasma-treating the etched layer.

8. A radiopaque stent graft comprising:
a stent graft (101) having a functionalized surface (107);
a tie layer (110) surrounding a functionalized surface (107);
an etched adherent layer covering at least a portion of the tie layer (110), wherein the etched adherent layer comprises a filled slot (119), and wherein the filled slot (119) comprises a radiopaque marker.

9. The radiopaque stent graft of claim 8, further comprising a sealant layer (125) encapsulating the filled slot and/or wherein the sealant layer comprises a flexible acrylate adhesive.

10. The radiopaque stent graft of any of claims 8-9, wherein the base stent graft (101) comprises polytetrafluoroethylene.

11. The radiopaque stent graft of any of claims 8-10, wherein the etched layer further comprises a poly(p-xylylene), wherein the poly(p-xylylene) preferably comprises one or more of parylene C, parylene N, parylene D, parylene X, parylene AF-4, parylene SF, parylene HT, parylene VT-4 (parylene F), parylene CF, parylene A, or parylene AM.

12. The radiopaque stent graft of any of claims 8-11, further comprising one or more additional filled slots, wherein the one or more additional filled slots each comprise a radiopaque marker.

13. The radiopaque stent graft of any of claims 8-12, wherein the radiopaque marker comprises a radiopaque material.

14. The radiopaque stent graft of any of claims 8-13, wherein the etched layer is functionalized to securely affix the radiopaque marker to the slot (119).

15. The radiopaque stent graft of any of claims 8-14, wherein the tie layer (110) has a thickness of from 1nm to 100 nm (10 angstroms to 1,000 angstroms).

## Patentansprüche

1. Verfahren zur Herstellung eines röntgendichten Stentgrafts (100), umfassend:
Behandeln einer äußeren Oberfläche eines Basisstentgrafts (101), um eine funktionalisierte Oberfläche (107) herzustellen;
Anordnen einer Verbindungsschicht (110) über der funktionalisierten Oberfläche (107);
Anordnen einer Haftschicht (115) über der Verbindungsschicht (110);
Ätzen eines Schlitzes (119) in die Haftschicht (115), um eine geätzte Schicht herzustellen;
Behandeln der geätzten Schicht; und
Positionieren eines röntgendichten Markers in dem Schlitz (119) der geätzten Schicht, um einen gefüllten Schlitz herzustellen und dadurch den röntgendichten Stentgraft (100) herzustellen.

2. Verfahren nach Anspruch 1, wobei der Basisstentgraft (101) Polytetrafluorethylen umfasst.

3. Verfahren nach einem der vorstehenden Ansprüche, wobei die Haftschicht (115) ein Poly(p-xylylen) umfasst, wobei das Poly(p-xylylen) vorzugsweise eines oder mehrere von Parylen C, Parylen N, Parylen D, Parylen X, Parylen AF-4, Parylen SF, Parylen HT, Parylen VT-4 (Parylen F), Parylen CF, Parylen A oder Parylen AM umfasst.

4. Verfahren nach einem der vorstehenden Ansprüche, weiter umfassend das Beschichten des gefüllten Schlitzes mit einem Klebstoff, um eine Dichtungsschicht (125) herzustellen.

5. Verfahren nach einem der vorstehenden Ansprüche, weiter umfassend:
Ätzen eines oder mehrerer zusätzlicher Schlitze (119) in die Haftschicht;
Plasmabehandeln des einen oder der mehreren zusätzlichen Schlitze (119);
Positionieren eines röntgendichten Markers in jeden des einen oder der mehreren zusätzlichen Schlitze (119), um einen oder mehrere zusätzliche gefüllte Schlitze (119) herzustellen und dadurch den röntgendichten Stentgraft herzustellen, vorzugsweise weiter umfassend das Beschichten der gefüllten Schlitze mit einem Klebstoff, um eine Dichtungsschicht (125) herzustellen, wobei das Beschichten des einen oder der mehreren zusätzlichen gefüllten Schlitze (119) bevorzugter die Verwendung eines flexiblen Acrylatklebstoffs umfasst, weiter noch bevorzugter umfassend das Aushärten des Klebstoffs, um die Dichtungsschicht herzustellen.

6. Verfahren nach einem der vorstehenden Ansprüche, wobei das Ätzen ein Laserätzen umfasst und/oder wobei das Behandeln der äußeren Oberfläche eines Basisstentgrafts (101) ein Plasmabehandeln der äußeren Oberfläche umfasst.

7. Verfahren nach einem der vorstehenden Ansprüche, wobei das Behandeln der geätzten Schicht ein Plasmabehandeln der geätzten Schicht umfasst.

8. Röntgendichter Stentgraft, umfassend:
einen Stentgraft (101) mit einer funktionalisierten Oberfläche (107);
eine Verbindungsschicht (110), die eine funktionalisierte Oberfläche (107) umgibt;
eine geätzte Haftschicht,
die mindestens einen Teil der Verbindungsschicht (110) bedeckt, wobei die geätzte Haftschicht einen gefüllten Schlitz (119) umfasst, und wobei der gefüllte Schlitz (119) einen röntgendichten Marker umfasst.

9. Röntgendichter Stentgraft nach Anspruch 8, weiter umfassend eine Dichtungsschicht (125), die den gefüllten Schlitz einkapselt, und/oder wobei die Dichtungsschicht einen flexiblen Acrylatklebstoff umfasst.

10. Röntgendichter Stentgraft nach einem der Ansprüche 8 - 9, wobei der Basisstentgraft (101) Polytetrafluorethylen umfasst.

11. Röntgendichter Stentgraft nach einem der Ansprüche 8 - 10, wobei die geätzte Schicht weiter ein Poly(p-xylylen) umfasst, wobei das Poly(p-xylylen) vorzugsweise eines oder mehrere von Parylen C, Parylen N, Parylen D, Parylen X, Parylen AF-4, Parylen SF, Parylen HT, Parylen VT-4 (Parylen F), Parylen CF, Parylen A oder Parylen AM umfasst.

12. Röntgendichter Stentgraft nach einem der Ansprüche 8 - 11, weiter umfassend einen oder mehrere zusätzliche gefüllte Schlitze, wobei der eine oder die mehreren zusätzlichen gefüllten Schlitze jeweils einen röntgendichten Marker umfassen.

13. Röntgendichter Stentgraft nach einem der Ansprüche 8 - 12, wobei der röntgendichte Marker ein röntgendichtes Material umfasst.

14. Röntgendichter Stentgraft nach einem der Ansprüche 8 - 13, wobei die geätzte Schicht funktionalisiert ist, um den röntgendichten Marker sicher an dem Schlitz (119) zu befestigen.

15. Röntgendichter Stentgraft nach einem der Ansprüche 8 - 14, wobei die Verbindungsschicht (110) eine
Dicke von 1 nm bis 100 nm (10 Angström bis 1.000 Angström) aufweist.

## Revendications

1. Procédé de production d'un implant de type stent (100) radio-opaque comprenant :
le fait de traiter une surface extérieure d'un implant de type stent de base (101) de manière à former une surface fonctionnalisée (107) ;
le fait de disposer une couche de liaison (110) sur la surface fonctionnalisée (107) ;
le fait de disposer une couche adhérente (115) sur la couche de liaison (110) ;
le fait de graver une fente (119) dans la couche adhérente (115) de manière à former une couche gravée ;
le fait de traiter la couche gravée ; et
le fait de positionner un marqueur radio-opaque dans la fente (119) de la couche gravée dans le but d'obtenir une fente remplie et à produire ainsi l'implant de type stent (100) radio-opaque.

2. Procédé selon la revendication 1, dans lequel l'implant de type stent de base (101) comprend du polytétrafluoroéthylène.

3. Procédé selon une quelconque revendication précédente, dans lequel la couche adhérente (115) comprend un poly(p-xylylène), dans lequel le poly(p-xylylène) comprend de préférence un ou plusieurs parmi le parylène C, le parylène N, le parylène D, le parylène X, le parylène AF-4, le parylène SF, le parylène HT, le parylène VT-4 (parylène F), le parylène CF, le parylène A ou le parylène AM.

4. Procédé selon une quelconque revendication précédente, comprenant en outre le fait de revêtir la fente remplie avec un adhésif dans le but de former une couche d'étanchéité (125).

5. Procédé selon une quelconque revendication précédente, comprenant en outre :
le fait de graver une ou plusieurs fentes supplémentaires (119) dans la couche adhérente ;
le fait de traiter au plasma les une ou plusieurs fentes supplémentaires (119) ;
le fait de positionner un marqueur radio-opaque dans chacune des une ou plusieurs fentes supplémentaires (119) dans le but d'obtenir une ou plusieurs fentes remplies supplémentaires (119) et à produire ainsi l'implant de type stent radio-opaque, comprenant de préférence en outre le fait de revêtir les fentes remplies avec un adhésif dans le but de former une couche d'étanchéité (125), dans lequel le fait de revêtir les une ou plusieurs fentes remplies supplémentaires (119) comprend de préférence l'utilisation d'un adhésif acrylique souple, et de façon plus préférée comprend le durcissement de l'adhésif dans le but de former la couche d'étanchéité.

6. Procédé selon une quelconque revendication précédente, dans lequel la gravure comprend une gravure au laser et/ou dans lequel le traitement de la surface extérieure d'un implant de type stent de base (101) comprend le traitement au plasma de la surface extérieure.

7. Procédé selon une quelconque revendication précédente, dans lequel le traitement de la couche gravée comprend le traitement au plasma de la couche gravée.

8. Implant de type stent radio-opaque comprenant :
un implant de type stent (101) présentant une surface fonctionnalisée (107) ;
une couche de liaison (110) enveloppant la surface fonctionnalisée (107) ;
une couche adhérente gravée
recouvrant au moins une partie de la couche de liaison (110), dans lequel la couche adhérente gravée comprend une fente remplie (119), et dans lequel la fente remplie (119) comprend un marqueur radio-opaque.

9. Implant de type stent radio-opaque selon la revendication 8, comprenant en outre une couche d'étanchéité (125) encapsulant la fente remplie et/ou dans laquelle la couche d'étanchéité comprend un adhésif acrylique souple.

10. Implant de type stent radio-opaque selon l'une quelconque des revendications 8 à 9, dans laquelle l'implant de type stent de base (101) comprend du polytétrafluoroéthylène.

11. Implant de type stent radio-opaque selon l'une quelconque des revendications 8 à 10, dans laquelle la couche gravée comprend en outre un poly(p-xylylène), dans laquelle le poly(p-xylylène) comprend de préférence un ou plusieurs parmi le parylène C, le parylène N, le parylène D, le parylène X, le parylène AF-4, le parylène SF, le parylène HT, le parylène VT-4 (le parylène F), le parylène CF, le parylène A ou le parylène AM.

12. Implant de type stent radio-opaque selon l'une quelconque des revendications 8 à 11, comportant en outre une ou plusieurs fentes remplies supplémentaires, dans laquelle les une ou plusieurs fentes remplies supplémentaires comprennent chacune un marqueur radio-opaque.

13. Implant de type stent radio-opaque selon l'une quelconque des revendications 8 à 12, dans laquelle le marqueur radio-opaque comprend une matière radio-opaque.

14. Implant de type stent radio-opaque selon l'une quelconque des revendications 8 à 13, dans laquelle la couche gravée est fonctionnalisée dans le but de fixer solidement le marqueur radio-opaque à la fente (119).

15. Implant de type stent radio-opaque selon l'une quelconque des revendications 8 à 14, dans laquelle la couche de liaison (110) présente une épaisseur comprise entre 1 nm et 100 nm (10 angströms et 1000 angströms).
